# EUROPEAN PATENT APPLICATION

(11) **EP 4 660 289 A2**
(43) Date of publication of application: **10.12.2025**
(21) Application number: 24216321.0
(22) Date of filing: 28.11.2024
(51) Int. Cl.: C12M 1/00, C12M 3/00, C12M 1/12, C12N 5/071, G01N 33/50

(54) **CHIPS, SYSTEMS AND METHODS FOR PRODUCING VASCULARIZED TISSUES**

(30) Priority: 13.05.2024 TW 113117660
(71) Applicant: National Applied Research Laboratories, Taipei City 106 (TW); National Taiwan University, Taipei City 106 (TW)
(72) Inventor: Hsu, Yu-Hsiang, 106 Taipei City (TW); Lin, Yu-Zhou, 235 New Taipei City (TW); Chang, Kai-Chieh, 420 Taichung City (TW); Chin, Hsian-Jean, 11529 Taipei City (TW); Wang, Chi-Kuang, 11529 Taipei City (TW); Su, Yu-Chia, 11529 Taipei City (TW)
(74) Representative: Lang, Christian

(57) **Abstract**

Disclosed herein is a chip for producing vascularized tissues. According to some embodiments of the present disclosure, the chip comprises a loading chamber, a culture chamber, a fluid collection chamber, an inflow channel, at least one outflow channel, two side channels, two upstream reservoirs, and two downstream reservoirs. Also disclosed herein is a system comprising a pair of the present chips, and methods of producing vascularized tissues by using the present chip or system.

## Description

### BACKGROUND OF THE INVENTION

### 1. FIELD OF THE INVENTION

The present disclosure in general relates to the field of tissue culture. More particularly, the present disclosure relates to a chip and a system for producing vascularized tissues.

### 2. DESCRIPTION OF RELATED ART

During the past few decades, Organ-on-a-Chip (OoC) and Microphysiological system (MPS) have become an actively researched area that offer a promising alternative to conventional biochemical laboratory and animal testing. By miniaturizing the macroscopic biophysical and biochemical processes to a millimeter or sub-millimeter scale, OoC and MPS can simulate the main functional aspects of a living tissue or organ (*e.g.*, living organs, tissues and/or cells) and enable the integration of various assays onto a single chip. Nowadays, the OoC and MPS technologies have been widely used in the field of life science research and medical fields, such as disease diagnosis, therapeutics, drug screening, drug delivery, environmental testing, and tissue engineering.

OoC and MPS provide a platform to culture and analyze three-dimensional (3D) tissues. Compared to traditional two-dimensional (2D) cell/tissue cultures that lack spatial organization and cell-cell and cell-matrix interactions, 3D cell/tissue cultures impart tissuespecific architecture and complex cellular interactions, and provide a more accurate and representative model of *in vivo* conditions. However, current 3D cell/tissue cultures fail to recreate the biological microvasculature for nutrient delivery, oxygen transport, and metabolic waste removal. While some research attempted to integrate microfluidics as a synthetic capillary in the 3D cell/tissue cultures, it is still difficult to precisely mimic the dynamic 3D microenvironment found *in vivo* due to the lack of vasculatures to simulate the physiological mass transport in human tissues.

In view of the foregoing, there is a continuing interest in developing a novel system for culturing 3D tissues having vascular networks developed therein.

### SUMMARY

The following presents a summary of the disclosure. This summary is not an extensive overview of the disclosure, and it does not identify key/critical elements of the present invention or delineate the scope of the present invention. Its sole purpose is to present some concepts disclosed herein in a simplified form as a prelude to the more detailed description that is presented later.

As embodied and broadly described herein, the first aspect of the present disclosure is directed to a chip for producing a vascularized tissue from a tissue mixture. According to some embodiments of the present disclosure, the chip comprises,
a loading chamber;
a culture chamber disposed downstream to the loading chamber;
a fluid collection chamber disposed downstream to the culture chamber;
an inflow channel connecting the loading chamber and the culture chamber;
at least one outflow channel connecting the culture chamber and the fluid collection chamber;
a first and a second side channels disposed symmetrically on opposite sides of the culture chamber and connected thereto, wherein each side channel has an upstream end, a V-shape bent section and a downstream end;
two upstream reservoirs (*i.e.,* a first and a second upstream reservoirs) respectively connected to two upstream ends of the first and second side channels; and
two downstream reservoirs (*i.e.,* a first and a second downstream reservoirs) respectively shaped as funnels and connected to two downstream ends of the first and second side channels.

According to some exemplary embodiments of the present disclosure, the chip comprises three outflow channels.

In the embodiments of the present disclosure, each side channel is arranged in the manner that the V-shape bent section is connected to the culture chamber on one side, and the side of the V-shape bent section connected to the culture chamber has a plurality of pores disposed thereon. Preferably, the inner surface of the V-shape bent section of each side channel is coated with a hydrophobic material, while the inner surface of the rest of the side channel is coated with a hydrophilic material.

According to certain embodiments, each of the upstream reservoirs and downstream reservoirs is shaped as a funnel.

According to some embodiments, the inflow channel has an end shaped as a trapezoid toward the loading chamber and connected thereto.

According to some embodiments of the present disclosure, the tissue mixture comprises a 3D tissue, an endothelial cell, a stromal cell, and a hydrogel. In one embodiment of the present disclosure, the chip further comprises at least one obstructing block to prevent the 3D tissue from flowing into the outflow channel, in which the obstructing block is disposed of within the outflow channel and proximal to the culture chamber. Preferably, the obstructing block has a size smaller than the size of the 3D tissue. In another embodiment of the present disclosure, the inner dimension of a section of the outflow channel proximal to the culture chamber is smaller than that of the rest of the outflow channel, so as prevent the 3D tissue from flowing into the outflow channel. Preferably, the inner dimension of the section is smaller than the diameter or maximal length of the 3D tissue.

According to certain embodiments of the present disclosure, each of the pores disposed on the side of the V-shape bent section has a size in a range between 30 µm to 100 µm.

Depending on desired purpose, the hydrophobic material coated on the inner surface of the side channel is selected from the group consisting of polyepoxide, polyethylene (PE), polystyrene (PS), polyvinylchloride (PVC), polytetrafluorethylene (PTFE), polydimethylsiloxane (PDMS), polyester, polymethyl methacrylate (PMMA), polyurethane (PU), and a combination thereof; and the hydrophilic material coated on the inner surface of the side channel is selected from the group consisting of polyethylene glycol (PEG), oligoethylene glycol (OEG), polyacrylamide, polyacrylic acid (PAA), polyvinyl alcohol (PVA), polystyrene sulfonic acid (PSS), poly-lysine, and a combination thereof.

Preferably, the culture chamber is made of a material with low or poor gas permeability, for example, PMMA, PS, PE, PVC, PU, PAM, polypropylene (PP), polyethylene terephthalate (PET), or a combination thereof. The culture chamber can also be made of gas-permeable materials, for example, polydimethylsiloxane (PDMS) and thermoplastic polyurethane (TPU), and its surface is coated with low gas-permeable materials.

The second aspect of the present disclosure pertains to a system for producing a vascularized tissue from a tissue mixture (*e.g*., a tissue mixture comprising a 3D tissue, an endothelial cell, a stromal cell, and a hydrogel). The system comprises a tissue plate and a driving device coupled to the tissue plate. Specifically, the tissue plate comprises a pair of the present chips, and a linking portion disposed between the pair of chips. The driving device comprises a rod having two ends, an overtube, a plurality of fastening elements, and a driving element, in which the overtube is mounted on one end of the rod, and has a lumen for the tissue plate to pass therethrough; the fastening elements are inserted into the overtube and coupled to the linking portion of the tissue plate so as to removably fasten the tissue plate to the overtube; and the driving element is coupled to the other end of the rod for driving the tissue plate to rotate along the longitudinal axis of the rod.

Also disclosed herein is a method of producing a vascularized tissue by using the chip or system of the present disclosure. The method comprises,
(a) adding a tissue mixture into the loading chamber of the chip, wherein the tissue mixture comprises a 3D tissue, an endothelial cell, a stromal cell, and a hydrogel;
(b) adding an equal amount of a first medium into each of the two upstream reservoirs of the chip, wherein the first medium is free of vascular endothelial growth factor (VEGF) and basic fibroblast growth factor (bFGF);
(c) incubating the product of step (b) at 37°C for at least 3 days;
(d) adding an equal amount of an endothelial cell to each of the two upstream reservoirs of the chip;
(e) incubating the product of step (d) at 37°C for at least 3 hours; and
(f) adding an equal amount of a second medium into each of the two upstream reservoirs of the chip, wherein the second medium comprises the VEGF and bFGF.

Depending on the intended purpose, the 3D tissue of the tissue mixture may be a biological sample isolated from a subject or an artificial synthetic tissue.

According to one exemplary embodiment, in the tissue mixture, the endothelial cell is a human umbilical vein endothelial cell (HUVEC), the stromal cell is a fibroblast, and the hydrogel is a fibrin gel.

According to some preferred embodiments of the present disclosure, the volume ratio of the 3D tissue and the culture chamber of the chip is about 1 : 10 to 1 : 40.

Many of the attendant features and advantages of the present disclosure will become better understood with reference to the following detailed description considered in connection with the accompanying drawings.

### BRIEF DESCRIPTION OF THE DRAWINGS

The present description will be better understood from the following detailed description read in light of the accompanying drawings.
Figures 1A to 1C are schematic diagrams of chip 100 according to some embodiments of the present disclosure.
Figures 2A and 2B are schematic diagrams respectively depicting chip 200A and chip 200B according to various embodiments of the present disclosure.
Figure 3 is a schematic diagram of the inflow channel 320 according to some embodiments of the present disclosure. T: 3D tissue.
Figures 4A and 4B are schematic diagrams of system 400 according to some embodiments of the present disclosure.
Figures 5A and 5B are schematic diagrams of system 500 according to alternative embodiments of the present disclosure.

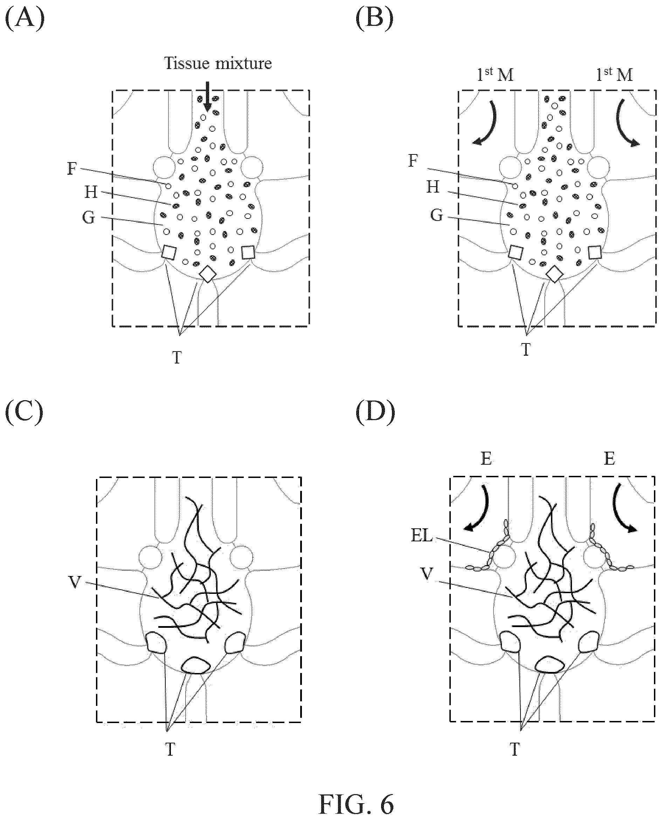

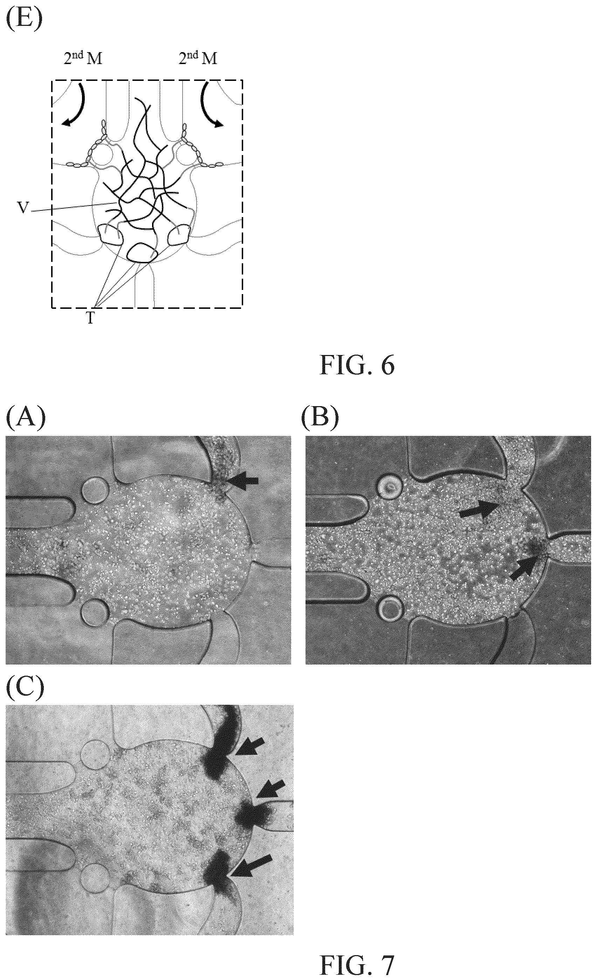

Figure 6 depicts schematic diagrams of the production of the vascularized tissue from a tissue mixture according to one embodiment of the present disclosure. F: fibroblast; H: HUVEC; G: hydrogel; T: 3D tissue; V: neovessels (vasculogenesis); EL: endothelial layer. The black arrow in Panel (A) indicates the flow direction of the tissue mixture from the inflow channel into the culture chamber; the black arrows in Panels (B) and (E) respectively indicate the flow directions of the first medium (1^{st} M) and second medium (2^{nd} M) in side channels; the black arrows in Panel (D) indicate the flow directions of endothelial cells (E) in side channels.
Figure 7 depicts photograms of the present chip containing one 3D tissue (Panel (A)), two 3D tissues (Panel (B)), or three 3D tissue (Panel (C)) in its culture chamber according to Example 1 of the present disclosure. The black arrows indicate the 3D tissues.
Figure 8 is the result of the finite element analysis (FEA) that depicts the pressure gradient in the culture chamber of the present chip in the presence of one 3D tissue (Panel (A)), two 3D tissues (Panel (B)), or three 3D tissue (Panel (C)) according to Example 1 of the present disclosure.
Figure 9 is the result of FEA that depicts the mass-transfer rate in the culture chamber of the present chip in the presence of one 3D tissue (Panel (A)), two 3D tissues (Panel (B)), or three 3D tissue (Panel (C)) according to Example 1 of the present disclosure.
Figures 10A and 10B are results of FEA respectively depicting the distributions of cytokines and morphogen released from the 3D tissue(s) in the culture chamber in the presence of one 3D tissue (Panel (A)), two 3D tissues (Panel (B)), or three 3D tissue (Panel (C)) according to Example 1 of the present disclosure. Figure 10A: The tissue mixture was incubated at 37°C for 12 hours. Figure 10B: The tissue mixture was incubated at 37°C for 24 hours.
Figure 11A and 11B are photographs respectively depicting the vascular networks according to Example 2 of the present disclosure, in which the vascular network of Figure 11A was formed via mixing HUVEC, NHLF and fibrin gel, and the vascular network of Figure 11B was formed via mixing liver-tumor patient-derived xenograft, HUVEC, NHLF, and fibrin gel.
Figure 12 is a photograph of the vascularized tissue treated with chemotherapeutic drug FOLFOX according to Example 3 of the present disclosure. The 3D tumor region (PDX) in the vascularized tissue was boxed by dash lines.
Figure 13 is a photograph of the vascularized tissue treated with chimeric antigen receptor T (CAR-T) according to Example 3 of the present disclosure. The 3D tumor region (PDX) and CAR-T cells in the vascularized tissue were respectively boxed and indicated.

In accordance with common practice, the various described features/elements are not drawn to scale but instead are drawn to best illustrate specific features/elements relevant to the present invention. Also, reference numerals and designations in the various drawings are used to indicate elements/parts.

### DETAILED DESCRIPTION OF THE INVENTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

### I. DEFINITION

For convenience, certain terms employed in the specification, examples and appended claims are collected here. Unless otherwise defined herein, scientific and technical terminologies employed in the present disclosure shall have meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

As used herein, the term "three-dimensional tissue" or "3D tissue" refers to an aggregate of cells in which the cells are three-dimensionally arranged through extracellular matrix components. A three-dimensional tissue may be a biological sample isolated from a subject (*e.g.,* a human sample, a murine sample, or a xenograft sample), or an artificial synthetic tissue (*e.g.,* a three-dimensional assembly including one or more types of cells artificially produced through cell culture or tissue engineering; for example, organoid, cell spheroid, tumor spheroid, and etc.). According to some exemplary embodiments, the three-dimensional tissue is a patient-derived xenograft (PDX), *i.e.,* a cancerous tissue derived from a patient's tumor (such as a liver tumor) and implanted directly into an immune-deficient animal (*e.g.,* a mouse). In one specific embodiment, the three-dimensional tissue is a liver-tumor patient-derived xenograft. The shape of the three-dimensional tissue is not particularly limited, and examples thereof include a spherical shape, an ellipsoidal shape, and a cuboid shape.

As used herein, the term "inner dimension" refers to the span from one interior surface (the first interior surface) of a pipe or tube (*e.g.,* the outflow channel of the present chip) to its opposite interior surface (the second interior surface, in which the first and second interior surfaces are spaced apart by an angle of 180 degrees) of the pipe or tube. Depending on intended purpose, the tube may have a rectangular, square, round or oval cross-section. According to certain embodiments of the present disclosure, the outflow channel has a rectangular cross-section; in this case, the term "inner dimension" refers to the inner height or width of the outflow channel. According to some embodiments of the present disclosure, the outflow channel has an square cross-section; in this case, the term "inner dimension" refers to the maximum inner width of the outflow channel. According to some alternative embodiments, the outflow channel has a round or oval cross-section; in this case, the term "inner dimension" refers to the inner diameter or maximum inner diameter of the outflow channel.

As used herein, the term "xenograft" is synonymous with the term "heterograft" and refers to a graft transferred from an animal of one species to one of another species. According to some embodiments of the present disclosure, the term "patient-derived xenograft" (PDX) refers to a cancerous tissue derived/isolated from a human patient's tumor (such as a liver tumor) and implanted directly into an immune-deficient animal (*e.g.,* a mouse).

The term "endothelial cell" is commonly understood by one of ordinary skill in the art and refers to a multifunctional cell type that forms the inner layer of body cavities, blood vessels (including arteries, veins and capillaries), and lymph vessels. According to one exemplary embodiment of the present disclosure, the endothelial cell is HUVEC. According to another exemplary embodiment of the present disclosure, the endothelial cell is Human umbilical artery endothelial cell (HUAEC).

The term "stromal cell" refers to a pluripotent cell capable of developing specifically into distinct types of connective tissue cells within the endoderm, mesoderm, or ectoderm. As known by one of ordinary skill in the art, stromal cells provide structure support for an organ, and produce extracellular matrix (ECM) proteins and basement membrane components. The term "stromal cell" as used herein is intended to mean a cell defined by its ability to adhere and proliferate in tissue-culture in the presence or absence of other cells and/or elements found in loose tissue. Examples of stromal cells suitable for use in the present invention include, but are not limited to, fibroblasts, smooth muscle cells, pericytes, and a combination thereof. According to one exemplary embodiment of the present disclosure, the stromal cell is a fibroblast (*e.g.*, human-lung fibroblast, NHLF). According to another exemplary embodiment of the present disclosure, the stromal cell is a mixture of fibroblasts, smooth muscle cell, and/or pericytes.

The term "hydrogel" is used in the broadest sense and refers to a three-dimensional, hydrophilic or amphiphilic polymeric network capable of taking up large quantities of water. The networks are composed of homopolymers or copolymers, and are insoluble due to the presence of covalent chemical or physical (ionic, hydrophobic interactions, entanglements) crosslinks. The crosslinks provide the network structure and physical integrity. Hydrogels exhibit a thermodynamic compatibility with water that allow them to swell in aqueous media. Examples of hydrogel suitable for use in the present invention include, but are not limited to, fibrin gel (*i.e.,* the hydrogel produced by fibrinogen and/or thrombin), collagen gel, hyaluronic acid gel, chitosan hydrogel, gelatin gel, MATRIGEL^{®}, and a combination thereof. Alternatively, the hydrogel may be produced from synthetic polymers, for example, PVA, PEG, polyacrylamide, poly (ethylene oxide), or a combination thereof. The method and procedures for producing hydrogels are known by a skill artisan; for example, see, US11241517B2; F. Ahmadi et al., Res Pharm Sci. (2015), 10(1): 1-16. Hence, the detailed description thereof is omitted herein for the sake of brevity. According to one exemplary embodiment of the present disclosure, the hydrogel is a fibrin gel.

As used herein, the term "longitudinal axis" refers to the axis running along the length and through the center of the referenced object (*i.e.,* the rod of the present system).

The term "subject" or "patient" refers to an animal including the human species from which a biological sample is isolated/derived. The term "subject" is intended to refer to both the male and female gender unless one gender is specifically indicated.

### II. DESCRIPTION OF THE INVENTION

The present disclosure aims at providing a novel chip for producing vascularized tissue from a 3D tissue. Depending on the intended purpose, the 3D tissue may be a biological sample isolated from a subject (*e.g*., a healthy subject or a cancer patient) or an artificial synthetic tissue. According to some exemplary embodiments of the present disclosure, the 3D tissue is a patient-derived xenograft (for example, liver-tumor patient-derived xenograft) that maintains the characteristics of the parental tumor, including the 3D structure, cell components, ECM scaffold, and immunity/immune cells. In these embodiments, a cancerous tissue (e.g., liver tumor) isolated/derived from a human patient is implanted into an immune-deficient animal (e.g., a mouse), so as to produce a 3D tumor tissue. Optionally, the cancerous tissue is cut into pieces (each piece has a diameter or maximal length of about 10 to 1,000 µm; for example, a cuboid with 150 µm or 200 µm on each side) with or without liquid nitrogen cryopreservation prior to the implantation.

### (i) Chips for producing vascularized tissues

The first aspect of the present disclosure is thus directed to a chip for producing a vascularized tissue from a tissue mixture. In addition to the 3D tissue (*e.g*., patient-derived xenograft) as described above, the tissue mixture further comprises an endothelial cell (*e.g*., HUVEC), a stromal cell (*e.g.,* fibroblast) and a hydrogel (*e.g*., fibrin gel) supporting neovessel formation (vasculogenesis) *in vitro.* According to some embodiments of the present disclosure, the instant chip is characterized by allowing incubation of the 3D tissue in one space of the culture chamber, while driving neovessel formation in another space of the culture chamber, and the two processes (*i.e.,* 3D-tissue culture and vasculogenesis processes) do not interfere with each other. Once the neovessel is partially formed, suitable angiogenic factors (*e.g*., VEGF and/or bFGF) and endothelial cells are added to the two side channels of the chip to create a monolayer of endothelial cells on the exposed hydrogel at the pores that connect the culture chamber and the two side channels. This step promotes vessel sprouting into the culture chamber and makes a connection with the neovessel inside the culture chamber (*i.e.,* angiogenesis) and subsequently link to the 3D tissue, thereby forming the vascularized tissue.

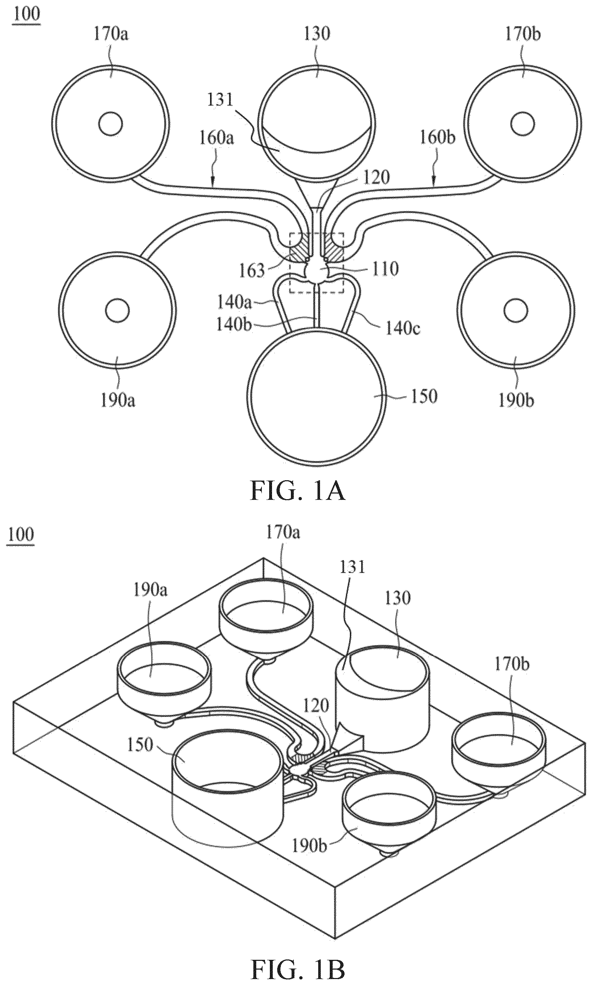

Reference is now made to Figures 1A and 1B, which are the top and side views of chip 100 according to one embodiment of the present disclosure. As depicted, the chip 100 comprises:
a culture chamber 110;
a loading chamber 130 disposed upstream to the culture chamber 110;
a fluid collection chamber 150 disposed downstream to the culture chamber 110;
an inflow channel 120 connecting the culture chamber 110 and the loading chamber 130;
three outflow channels 140a, 140b, 140c connecting the culture chamber 110 and the fluid collection chamber 150;
two upstream reservoirs 170a, 170b;
two downstream reservoirs 190a, 190b; and
two side channels 160a, 160b disposed symmetrically on opposite sides of the culture chamber 110 and connected thereto, in which the upstream and downstream ends of the side channel 160a are respectively connected to the upstream reservoir 170a and the downstream reservoir 190a, and the upstream and downstream ends of the side channel 160b are respectively connected to the upstream reservoir 170b and the downstream reservoir 190b.

Optionally, the chip 100 further comprises a lid (*e.g*., a half-moon lid 131 as exemplified in Figures 1A and 1B) covering the loading chamber 130. Preferably, the lid is placed on the side of the inflow channel 120 and the culture chamber 110 (Figures 1A and 1B).

Each of the upstream reservoirs 170a, 170b and downstream reservoirs 190a, 190b is shaped as a funnel (*i.e.,* with a wide, circular open top and a narrow open bottom) as illustrated in Figure 1B.

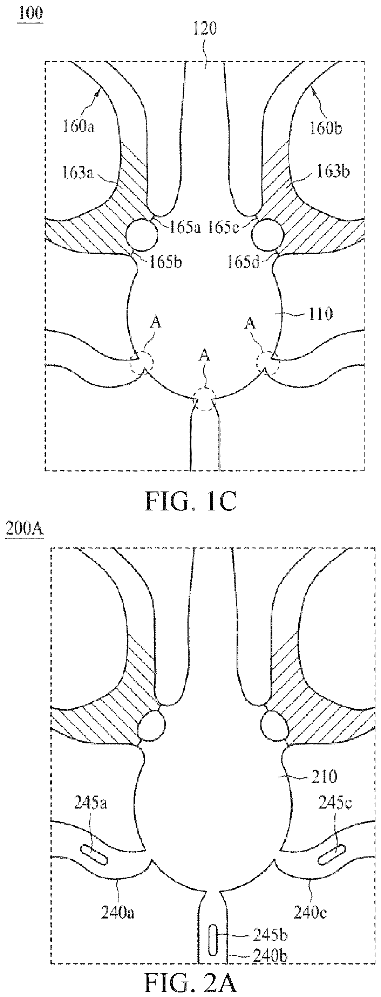

Each of the side channels 160a, 160b is characterized by having a V-shape bent section 163 that is connected to the culture chamber 110 on one side. Reference is further made to Figure 1C, which is a partial enlargement view of the chip 100. As illustrated, the side channels 160a, 160b are respectively connected to the culture chamber 110 via the V-shape bent sections 163a, 163b, in which the side of each side channels 160a, 160b connected to the culture chamber 110 has a plurality of pores disposed thereon. In this case, the side channel 160a is in fluid communication with the culture chamber 110 via two pores 165a, 165b disposed on one side of the V-shape bent section 163a, and the side channel 160b is in fluid communication with the culture chamber 110 via the two pores 165c, 165d disposed on one side of the V-shape bent section 163b.

Note that only two pores are illustrated in the exemplary embodiment depicted in Figure 1C, however, the one side of the V-shape bent section may comprise one or more than two pores (e.g., 3, 4, 5, 6, 7, or more pores), depending on the needs of the intended purpose. According to some embodiments of the present disclosure, the number of the pore controls the level of nutrition and oxygen supply and also the level of metabolite exchange. As could be appreciated, a skilled artisan may adjust the number and/or size of the pore on the V-shape bent section of each side channel in accordance with practical needs. For example, the side channel may have 1, 2, 3, 4, 5 or more pores on the side connected to the culture chamber, in which each pore has a size (diameter) ranging from 10 µm to 100 µm (*e.g.,* 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 µm). Preferably, the pore has a size smaller than that of a cell so as to prevent the backflow of cells into the side channel.

According to some embodiments of the present disclosure, the inner surface of the V-shape bent section of each side channel is coated with a hydrophobic material, while the inner surface of the rest of the side channel is coated with a hydrophilic material. Examples of the hydrophobic material suitable for use in the present chip include but are not limited to, PMMA, polyepoxide, polyester, PE, PS, PVC, PTFE, PDMS, PMMA, PU, and a combination thereof. Non-limiting examples of the hydrophilic material suitable for use in the present chip include, PEG, OEG, PAM, PAA, PVA, PSS, poly-lysine, and a combination thereof. The hydrophilic-hydrophobic-hydrophilic inner surface of the side channel allows that a medium (*e.g*., a culture medium) added to the upstream reservoir would rapidly flow into the upstream section (*i.e.,* the first hydrophilic section) of the side channel, followed by filling the 163a and 163b section that connects to the culture chamber with minimized bubble formation at the pore regions via slowly passing through the pore of the hydrophobic V-shape bent section, and then rapidly flowing into the downstream section (*i.e.,* the second hydrophilic section) of the side channel.

Preferably, the culture chamber of the present chip is made of a material with low or poor gas permeability, for example, PMMA, PS, PE, PVC, PU, PAM, PP, PET, or a combination thereof. The low/poor gas permeability of the material ensures that the oxygen in the culture chamber is mainly supplied through the side channels.

The above description is merely to exemplify the configuration of the chip structure; it should be understood that the scope of the present disclosure is not limited thereto. For example, the chip of the present invention may comprise 2, 3, 4, 5 or more outflow channels, and/or more than one fluid collection chamber.

In practice, the tissue mixture and the medium are respectively added to the loading chamber and upstream reservoirs that then flow into and mix in the culture chamber. To prevent the 3D tissue of the tissue mixture from flowing into the outflow channel, the chip of the present disclosure has a narrowed opening at the three outflow channels A to capture 3D tissues at these locations, as depicted in Fig. 1C. In another embodiment of the present invention, the outflow channel may further comprise an obstructing block disposed within the outflow channel. Reference is now made to Figure 2A, where a schematic view of the chip 200A having a plurality of obstructing blocks are depicted. Compared to the chip 100 of Figure 1C, the chip 200A of Figure 2A is characterized by having three obstructing blocks, *i.e.,* obstructing blocks 245a, 245b, 245c, respectively disposed within the outflow channels 240a, 240b, 240c. Preferably, the obstructing blocks 245a, 245b, 245c are respectively disposed proximal to the culture chamber 210 as depicted in Figure 2A. According to some exemplary embodiments of the present disclosure, each of the obstructing blocks 245a, 245b, 245c has a size smaller than the size of the 3D tissue. As could be appreciated, the size, number and/or position of the obstructing block may vary with intended purpose; for example, each of the outflow channels 240a, 240b, 240c may independently have 1, 2, 3 or more obstructing blocks disposed therein, in which the sizes of each obstructing blocks may be the same or different.

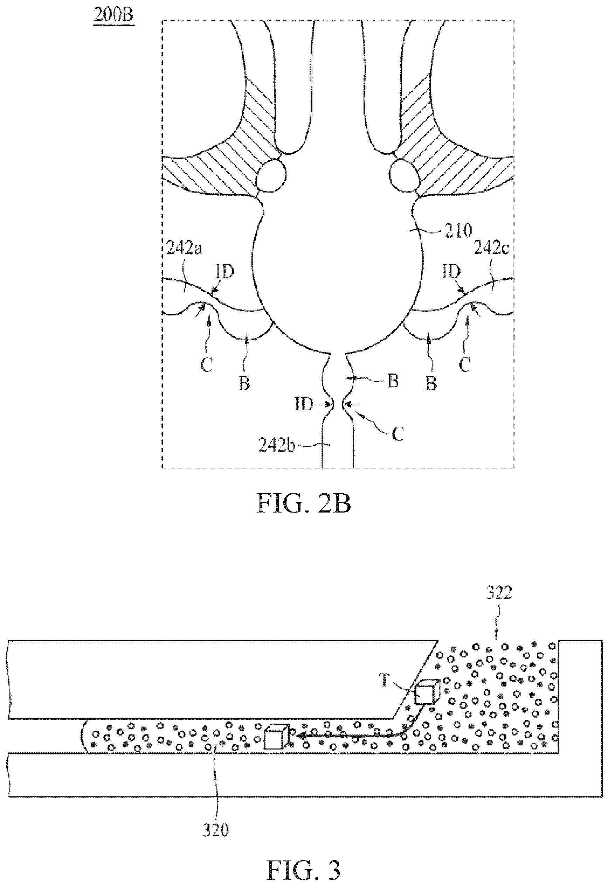

Additionally or alternatively, the outflow channel of the present chip may have a section with a narrow inner dimension to prevent the 3D tissue from flowing into the outflow channel. Figure 2B provides an alternative embodiment of the present chip, in which the chip 200B is quite similar to that of chip 100 of Figure 1C, except each of the outflow channels 242a, 242b, 242c has a concave portion (C) proximal to the culture chamber 210, in which the inner dimension (ID) of this portion (C) is smaller than that of the rest of the outflow channel, and/or is smaller than the diameter (preferably, the maximal diameter or length) of the 3D tissue so as to capture/keep the 3D tissue in the cavity at entrance of the outflow channels B. Note that the term "concave portion" refers to the outer shape of the particular portion of the outflow channel proximal to the culture chamber 210.

Reference is further made to Figure 3, which is the side view of the inflow channel 320 according to some optional embodiments of the present disclosure. As depicted, the inflow channel 320 has an end 322 shaped as a trapezoid toward the loading chamber and connected thereto. Note that such trapezoid design ensures that the 3D tissue (T) flows smoothly from the loading chamber into the flow channel 320, and/or that the large-size 3D tissue is pushed into the culture chamber.

### (ii) Systems for producing vascularized tissues

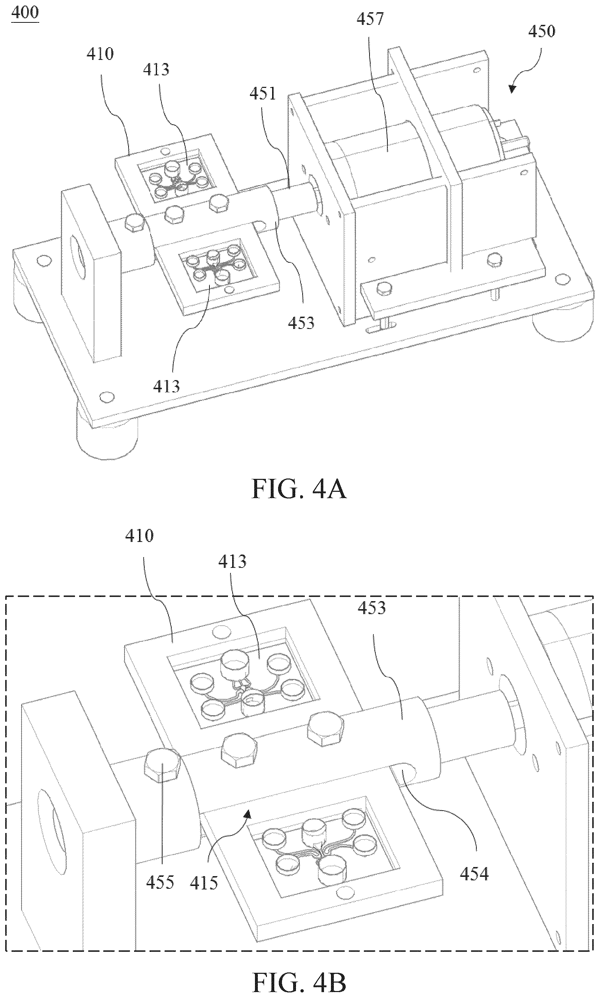

The second aspect of the present disclosure pertains to a system for producing vascularized tissues. Reference is made to Figures 4A and 4B, which are side view and partial enlargement view of a system 400 according to one embodiment of the present disclosure. Structurally, the system 400 comprises a tissue plate 410 and a driving device 450 coupling together. The tissue plate 410 comprises two chips 413, each chip may have a configuration as depicted in Figures 1 or 2; and a linking portion 415 disposed between the two chips 413. The driving device 450 comprises in its structure, a rod 451 having two ends, an overtube 453, a plurality of fastening elements 455, and a driving element 457. As depicted in Figures 4A and 4B, the overtube 453 is mounted on one end of the rod 451, and has a lumen 454 large enough to permit the passage of the tissue plate 410. The plurality of fastening elements 455 are inserted into the overtube 453 and coupled to the linking portion 415 of the tissue plate 410 so as to removably fasten the tissue plate 410 to the overtube 453. The driving element 457 is coupled to the other end of the rod 451 thereby driving the tissue plate 410 to rotate along the longitudinal axis of the rod 451. The direction of these two chips is the loading chamber is placed near the center, and thus, the thus-generated centrifugal force allows the tissue mixture, cells and/or medium in the loading chamber and/or upstream reservoirs to be drawn into the culture chamber, and/or allows the 3D tissue to be captured/kept at the bottom (*i.e.,* the downstream side connected to the outflow channels) of the culture chamber. The endothelial cells and stromal cells are uniformly distributed through the culture chamber. The lid 131 (*e.g*., the half-moon lid as depicted in Figures 1A and 1B) is used to prevent tissue mixture spilt out during the rotational loading process.

The fastening element 455 may be a threaded stud, a screw, a bolt, a nut, a rivet, a clip, a nail, or any known element for tightening or clamping two parts together.

The driving element 457 may be an electric motor, a fluid motor, a gasoline engine, an internal combustion engine, or any electromagnetic means with a rotating motor means. A skilled artisan may choose a suitable driving element in accordance with practical needs for the rotation purpose. According to some exemplary embodiments, the driving element 457 of the present chip is a servo motor controlled by a pulse width modulation (PWM).

As could be appreciated, the rotational speed of the present chip and the implantations of the tissue mixture, medium and/or other substances (*e.g.*, endothelial cells, testing drugs or agents) into the culture chamber are controlled by the driving element, accordingly, a skilled artisan may adjust the driving element by altering the rotational speed and the loading rate of substances. According to certain exemplary embodiments, the driving element starts from static state and may be accelerated with an angular acceleration of about 100 rad/s² to 1,000 rad/s² (*e.g.,* 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990 or 1,000 rad/s²); preferably, for the fibrin gel the driving element may start at an angular acceleration of about 200 rad/s² to 900 rad/s²; more preferably, the driving element may start at an angular acceleration of about 250 rad/s² to 800 rad/s². The substance implantation is carried out at a highest rotational speed ranging from about 1,000 rpm to 2,000 rpm (*e.g.*, 1,000, 1,010, 1,020, 1,030, 1,040, 1,050, 1,060, 1,070, 1,080, 1,090, 1,100, 1,110, 1,120, 1,130, 1,140, 1,150, 1,160, 1,170, 1,180, 1,190, 1,200, 1,210, 1,220, 1,230, 1,240, 1,250, 1,260, 1,270, 1,280, 1,290, 1,300, 1,310, 1,320, 1,330, 1,340, 1,350, 1,360, 1,370, 1,380, 1,390, 1,400, 1,410, 1,420, 1,430, 1,440, 1,450, 1,460, 1,470, 1,480, 1,490, 1,500, 1,510, 1,520, 1,530, 1,540, 1,550, 1,560, 1,570, 1,580, 1,590, 1,600, 1,610, 1,620, 1,630, 1,640, 1,650, 1,660, 1,670, 1,680, 1,690, 1,700, 1,710, 1,720, 1,730, 1,740, 1,750, 1,760, 1,770, 1,780, 1,790, 1,800, 1,810, 1,820, 1,830, 1,840, 1,850, 1,860, 1,870, 1,880, 1,890, 1,900, 1,910, 1,920, 1,930, 1,940, 1,950, 1,960, 1,970, 1,980, 1,990, or 2,000 rpm; preferably, about 1,200 rpm to 1,800 rpm; more preferably, about 1,300 rpm to 1,700 rmp) for about 100 ms to 1,000 ms (preferably, about 200 ms to 800 ms; more preferably, about 250 ms to 800 ms) with a deceleration time ranging about 100 ms to 1,000 ms (preferably, about 200 ms to 800 ms; more preferably, about 200 ms to 600 ms). According to various embodiments of the present disclosure, the driving element may start at an angular acceleration of 280 rad/s² to 800 rad/s², and the substance implantation is carried out at a highest rotational speed of 1,350 rpm to 1,620 rmp for 300 ms to 750 ms with a deceleration time of 200 ms to 500 ms. The aforementioned driving parameters of the driving element may be adjusted according to different hydrogel, which depends on its initial viscosity and the time to gelations.

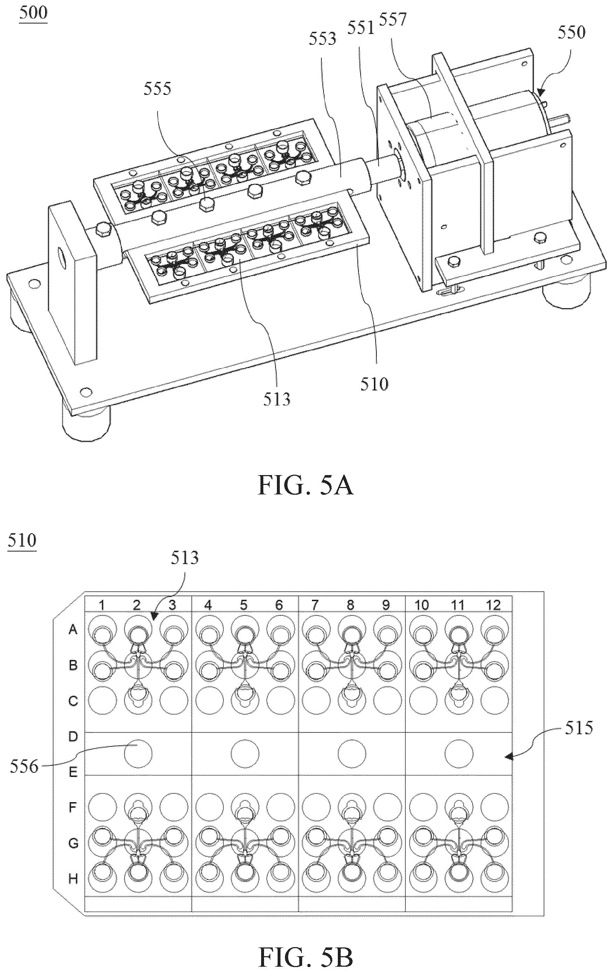

Figure 5A provides an alternative embodiment of the present system 500, the configuration of which is quite similar to that of Figure 4A, except eight chips 513 are included in the system 500. Similarly, the system 500 comprises a tissue plate 510 and a driving device 550, in which the tissue plate 510 comprises eight chips 513, which may have a configuration as depicted in any one of the chips in Figures 1 or 2. The driving device 550 comprises a rod 551, an overtube 553 mounted on one end of the rod 551, a plurality of fastening elements 555 inserted into the overtube 553 for fastening the tissue plate 510, and a driving element 557 coupled to the other end of the rod 551 for driving the tissue plate 510 to rotate along the longitudinal axis of the rod 551.

Figure 5B is the top view of the tissue plate 510 according to one embodiment of the present disclosure. As depicted, the tissue plate 510 comprises eight chips 513 disposed symmetrically on opposite sides of the linking portion 515, and four holes 556 disposed on the linking portion 515 for coupling with the fastening elements 555. As could be appreciated, each chip 513 may be independently inserted into the tissue plate 510 and/or independently dissembled from the tissue plate 510.

### (iii) Methods of producing vascularized tissues by using the present chips of systems

The third aspect of the present disclosure provides a method of producing a vascularized tissue by using the chip or system in accordance with any aspects, embodiments, and examples of the present disclosure. Reference is now made to Figure 6, which provides a schematic diagram depicting the production of the vascularized tissue from a tissue mixture. According to certain embodiments of the present disclosure, the method comprises,
(a) adding a tissue mixture into the loading chamber of the chip;
(b) adding an equal amount of a first medium into each of the two upstream reservoirs, wherein the first medium is free of an angiogenic factor;
(c) incubating the product of step (b) at 37°C for at least 3 days;
(d) adding an equal amount of an endothelial cell to each of the two upstream reservoirs;
(e) incubating the product of step (d) at 37°C for at least 3 hours; and
(f) adding an equal amount of a second medium into each of the two upstream reservoirs, wherein the second medium comprises at least one angiogenic factor.

In step (a), a tissue mixture is added to the loading chamber. As described in Panel (A) of Figure 6, the tissue mixture flowing from the loading chamber into the culture chamber comprises three 3D tissues (*e.g*., patient-derived xenograft; abbreviated as "T" in Figure 6), an endothelial cell (*e.g*., HUVEC; abbreviated as "H" in Figure 6), a stromal cell (*e.g.,* fibroblast; abbreviated as "F" in Figure 6), and a hydrogel (*e.g*., fibrin gel; abbreviated as "G" in Figure 6). The addition of the tissue mixture into the loading chamber may be carried out by using a pipette, an electric pipette, a robotic arm, or a combination thereof. Alternatively, the tissue mixture may be loaded by the systems disclosed in Figure 4A or 5A.

According to some embodiments of the present disclosure, each 3D tissue has a diameter or maximal length of about 10 µm to 1,000 µm (*e.g.*, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 µm).

Preferably, the volume ratio of the 3D tissue and the culture chamber is about 1 : 10 to 1 : 40; for example, 1 : 10, 1 : 15, 1 : 20, 1 : 25, 1 : 30, 1 : 35, or 1 : 40.

Depending on intended purpose, the endothelial cell and stromal cell are present in the tissue mixture at a cell number ratio of 10 : 1 to 1 : 10 (e.g., 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, or 1 : 10). Preferably, the number ratio of the endothelial cell and stromal cell in the tissue mixture ranges from 2 :1 to 1 : 2. According to one specific embodiment of the present disclosure, the cell number ratio of the endothelial cell and stromal cell in the tissue mixture is about 1 : 1.

The hydrogel may be any agent known to provide the extracellular matrix network structure and physical integrity supporting cell adhesion and/or growth. For example, fibrin gel, collagen gel, hyaluronic acid gel, chitosan hydrogel, gelatin gel, MATRIGEL^{®}, PVA, PEG, polyacrylamide, poly (ethylene oxide), or a combination thereof. According to one exemplary embodiment of the present disclosure, the hydrogel is fibrin gel. A skilled artisan may choose a suitable hydrogel and/or adjust the concentration of the hydrogel in accordance with an intended purpose.

In step (b), a first medium (abbreviated as "1^{st} M" in Panel (B) of Figure 6) is added into the two upstream reservoirs that then flow into the side channels. The first medium is characterized by not containing any angiogenic factors, for example, VEGF and bFGF. As described above, the medium added into the upstream reservoirs flows into the side channels and enters the culture chamber through the pores of the V-shape bent sections of the side channels. Preferably, the first medium is added in two equal amounts into the two upstream reservoirs so as to form a very small pressure gradient in the culture chamber, in which the pressure gradually decreases from the upper portion (*i.e.,* the upstream side connected to the inflow channel and side channels) of the culture chamber to the lower portion (*i.e.,* the downstream side connected to the outflow channel(s)) of the culture chamber. The pressure gradient ensures that the 3D tissue would be cultured in a space different from the space where the neovessel formation occurs. According to one embodiment of the present disclosure, the pressure of each upstream end of the side channels is about 50 Pa, and the pressure of each downstream end of the side channels, the loading reservoir and the collection reservoir are all about 30 Pa thereby forming a pressure gradient in the culture chamber.

In step (c), the chip containing the tissue mixture and the first medium is incubated at 37°C for at least 3 days (*e.g.,* 3, 4, 5, 6, 7, or more days). As depicted in Panel (C) of Figure 6, the 3D tissue (T) is cultured in the lower portion of the culture chamber, and the neovessel structure (vasculogenesis; abbreviated as "V") developed from the endothelial cell and stromal cell is formed in the top and central portions of the culture chamber.

In steps (d) and (e), an endothelial cell (abbreviated as "E" in Panel (D) of Figure 6; e.g., HUVEC) is added to the two upstream reservoirs of the chip (step (d)), and then incubated at 37°C for at least 3 to 6 hours thereby forming an endothelial layer (EL) on the V-shape bent section of each side channel as depicted in Panel (D) of Figure 6. Then, the second media (2^{nd} M) is added in two equal amounts into the two upstream reservoirs and cultured for at least 2 days (*e.g.,* 2, 3, 4, 5, 6, 7, or more days) (step (e)), so that angiogenic vessels are sprouted from these EL and make connections with V

According to some embodiments of the present disclosure, the method further comprises inclining the chip prior to the step (e), so that the endothelial cell would be attached on the V-shape bent section. Depending on desired purposes, the chip may be kept at a tilted angle ranging from 45 degrees to 80 degrees, for example, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 degrees. According to some preferred embodiments, the chip is kept at a tilted angle of 70-75 degrees. In one specific embodiment, the chip is kept at a tilted angle of 72 degrees.

In step (f), a second medium (abbreviated as "2^{nd} M" in Panel (E) of Figure 6) is added into the two upstream reservoirs of the present chip, then flows into the reaction chamber through the pores of the V-shape bent sections of the side channels. According to some embodiments of the present disclosure, the second medium contains at least one angiogenic factor that promotes vessel sprouting from the neovessel (V) and linking to the 3D tissue, thereby forming the vascularized tissue as depicted in Panel (E) of Figure 6. According to some embodiments of the present disclosure, the second medium comprises VEGF and bFGF. Preferably, the second medium is added in two equal amounts into the two upstream reservoirs.

The thus-produced vascularized tissue is useful in identifying drugs for treatments, verifying drug toxicity, and/or conducting immunotherapies. For example, different amounts of an anti-cancer candidate drug may be respectively added to the two upstream reservoirs of the present chip with different pressures, which makes one side channel has a higher pressure than the other side channel. This application creates a pressure gradient across the culture chamber that drives the flow of the culture medium inside the vessels, which mimics the bloodstream flow for delivering the drugs to the subject 3D tissue. The anti-cancer efficacy and/or cytotoxicity of the candidate drug may be determined by measuring the morphological changes of the vascularized tumor and/or analyzing the sample buffer collected from the fluid collection chamber of the present chip.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent. All publications cited herein are hereby incorporated by reference in their entirety.

### EXAMPLE

### Example 1 3D tissue culture by using the present chip

A tissue mixture was prepared by mixing a dissected patient-derived liver-tumor xenograft, human umbilical vein endothelial cell (HUVEC, 2.5×10⁷ cells/mL), human lung fibroblast (NHLF, 2.5×10⁷ cells/mL), and fibrin gel (1 U/mL to 10 U/mL), in which the patient-derived liver-tumor xenograft had a diameter ranging from 150 µm to 250 µm.

The thus-produced tissue mixtures with one, two, or three dissected patient-derived liver tumor xenografts were subjected to the chip of the present invention. As the photographs depicted in Panels (A), (B) and (C) of Figure 7, the chip had three outflow channels connected to the bottom portion of the culture chamber, in which the section of each outflow channel proximal to the culture chamber was designed to have a small inner dimension to prevent the 3D tissue of the tissue mixture from flowing into the outflow channel. According to the results, the 3D tissue(s) was/were captured by the proximal section of the outflow channels (Panels (A) to (C) of Figure 7).

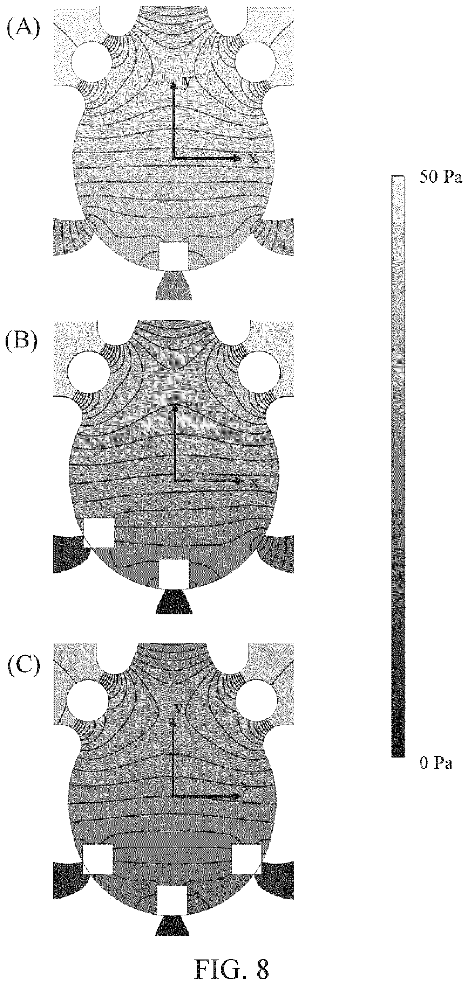

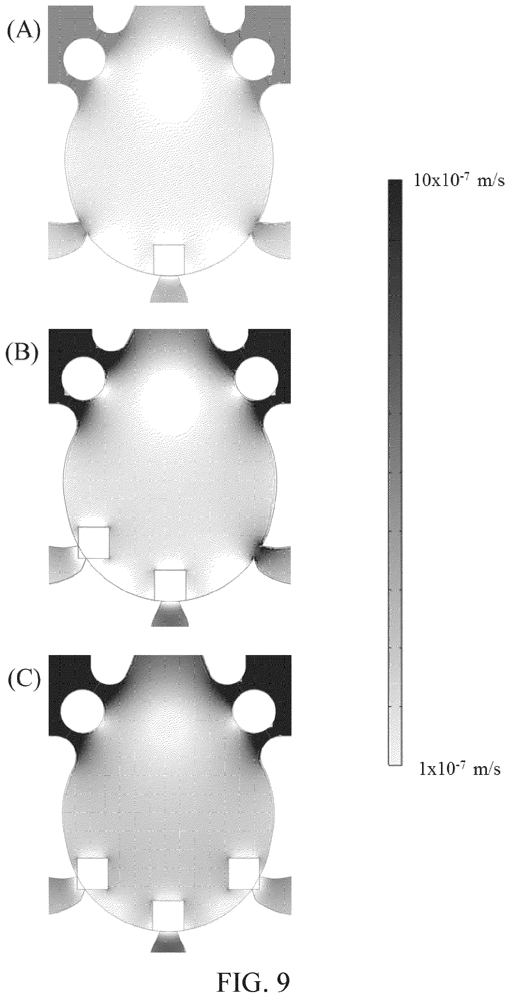

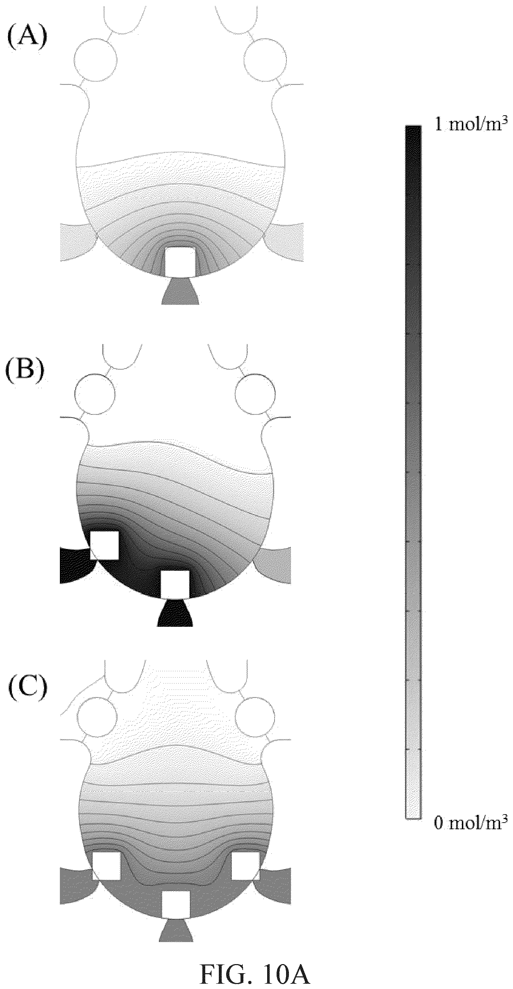

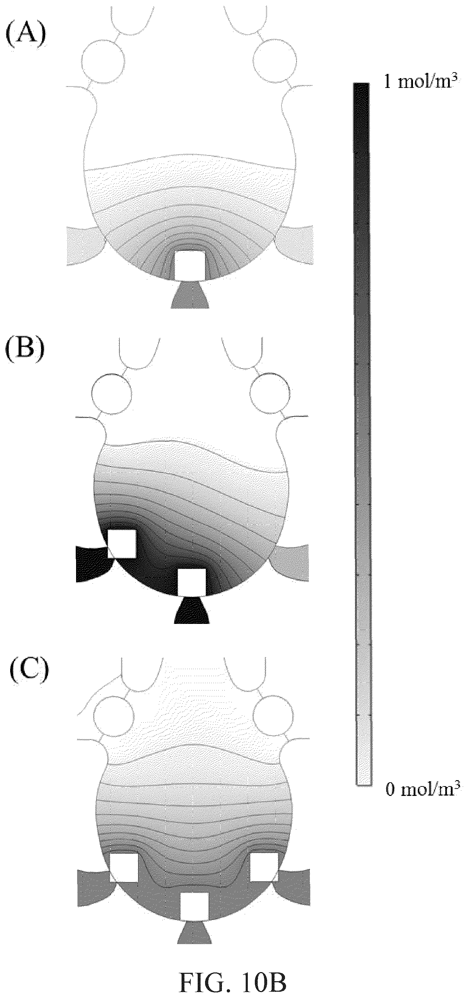

Then, a culture medium was added in two equal amounts into the upstream reservoirs of the present chip. According to the results analyzed by finite element analysis ( FEA), the addition of the culture medium created a pressure gradient in the culture chamber in the presence of one, two, or three 3D tissues, in which the pressure gradually decreased from the top portion to the bottom portion of the culture chamber (*i.e.,* decreasing along the y-axis of the culture chamber), and exhibited a symmetrical pattern on opposite sides of the culture chamber (*i.e.,* the pressures were symmetrically distributed with respect to the y-axis of the culture chamber) (Panels (A), (B) and (C) of Figure 8). Further, the data analyzed by FEA indicated that the mass-transfer rate in the culture chamber was lower than 5×10⁻⁷ m/s (Panels (A), (B) and (C) of Figure 9), demonstrating that the mass transfer occurred in the culture chamber via molecular diffusion, *i.e.,* the molecules in the culture chamber were transferred from a region of higher concentration to a region of lower concentration. The molecular diffusion facilitated the establishment of an ischemia and hypoxia condition in the culture chamber for inducing the vasculogenic and angiogenic processes of endothelial cells. The analytic results of FEA also indicated that the molecules released from the 3D tissue(s), such as cytokines and/or morphogen, were distributed around the 3D tissue (Panels (A), (B) and (C) of Figures 10A and 10B), minimizing the mutual interference between 3D tissue and neovessels. The data of Figures 10A and 10B was confirmed by a vascularized tissue established by liver-tumor patient-derived xenograft, in which the angiopoietin 2 (Ang-2) was distributed around the 3D tissue (data not shown).

### Example 2 Production of vascularized tissues

The mixture containing HUVEC (2.5×10⁷ cells/mL), NHLF (2.5×10⁷ cells/mL) and fibrin gel (1 U/mL to 10 U/mL) in the absence or presence of liver-tumor patient-derived xenograft were subjected to the chip of the present invention. An EGM^{™}-2 endothelial cell culture medium free of VEGF and bFGF was added to the tissue mixture through the side channels of the present chip. After incubating at 37°C with 5% O₂ for 3 to 7 days to stimulate vasculogensis in the culture chamber to form vessel network, HUVECs were added to the two side channels, followed by incubating at 37°C with 5% O₂ for 3 to 6 hours. Then, an EGM^{™}-2 endothelial cell culture medium containing VEGF and bFGF was added to the two side channels to stimulate angiogenic sprouting into the culture chamber and make connection with vasculogenic vessel network. Then, vascularized tissues were developed.

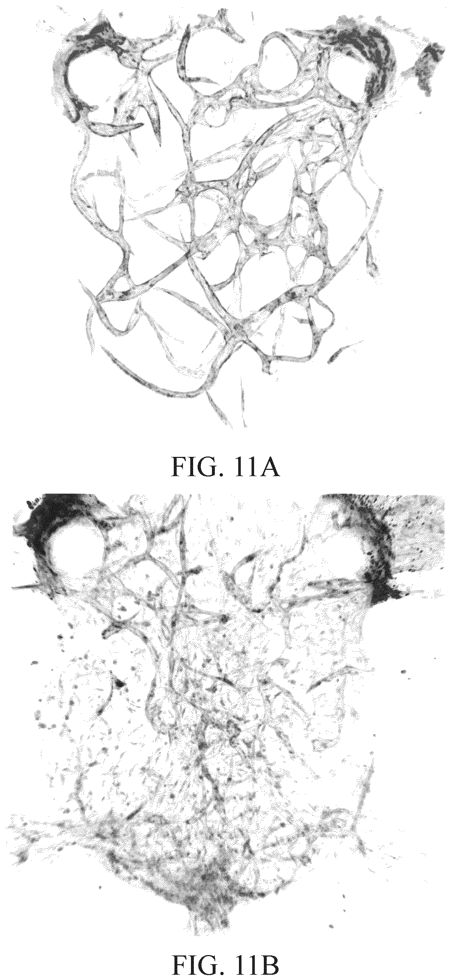

Figure 11A depicted the vascular network developed from the mixture of HUVEC, NHLF and fibrin gel, and Figure 11B depicted the vascular network developed from the mixture of patient-derived liver-tumor xenograft, HUVEC, NHLF and fibrin gel. These data demonstrated that the present chip is effective in producing vascularized normal and tumor tissues.

### Example 3 Uses of vascularized tissues in evaluating efficacy and anti-cancer drugs and immunotherapy

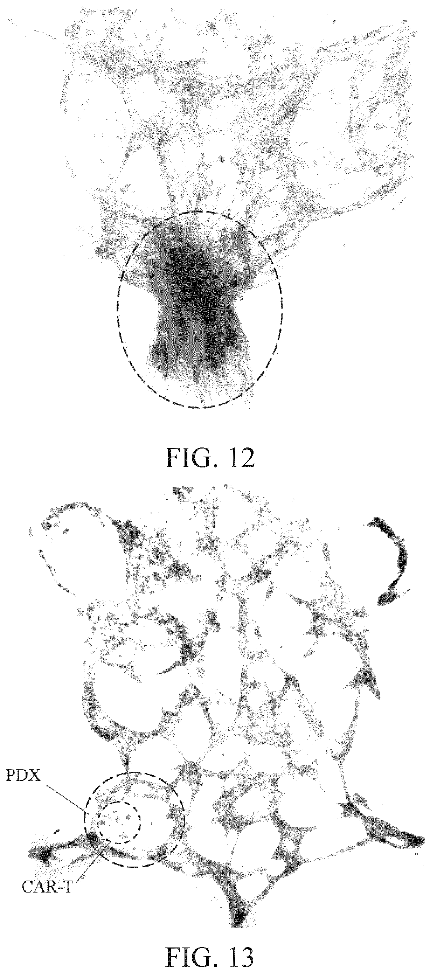

Different amounts of chemotherapeutic drug FOLFOX were respectively added to the upstream reservoirs of the present chip, creating a pressure gradient in the culture chamber that drives the culture medium flow in the vessel network to mimic the bloodstream in a subject. The data of Figure 12 depicted the image of tumor tissue (PDX) destroyed by FOLFOX (highlighted in dashed circle).

On the other hand, chimeric antigen receptor T (CAR-T) cells were added to the upstream reservoirs of the present chip to evaluate the anti-cancer effect of CAR-T cells. According to the data in Figure 13, the CAR-T cells were found in the tumor region (PDX) of the vascularized tissue, demonstrating that the CAR-T cells were capable of entering the tumor region (PDX) via the vascular network.

In conclusion, the present disclosure provides a novel chip to produce vascularized tissues from a 3D tissue (*e.g.*, patient-derived liver-tumor xenograft). According to the examples of the present disclosure, the present chip provides a pressure gradient in the culture chamber that allows the spatial separation of 3D tissue and neovessels, without interfering with each other. Once the neovessel formation is completed, the endothelial cells, and angiogenic factors VEGF and bFGF are added to the side channels to enable vessel sprouting from the endothelial layer on the side channels and connect to the vessel network of the 3D tissue inside the culture chamber, thereby forming vascularized tissues. The thus-produced vascularized tissues may serve as a tissue model for evaluating the efficacy and/or cytotoxicity of candidate drugs and immunotherapies.

It will be understood that the above description of embodiments is given by way of example only and that various modifications may be made by those with ordinary skill in the art. The above specifications, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. A chip for producing a vascularized tissue from a tissue mixture comprising a three-dimensional (3D) tissue, an endothelial cell, a stromal cell, and a hydrogel; the chip comprises,
a loading chamber;
a culture chamber disposed downstream to the loading chamber;
a fluid collection chamber disposed downstream to the culture chamber;
an inflow channel connecting the loading chamber and the culture chamber;
at least one outflow channel connecting the culture chamber and the fluid collection chamber;
a first and a second side channels disposed symmetrically on opposite sides of the culture chamber and connected thereto, wherein
each side channel has an upstream end, a V-shape bent section and a downstream end, and is arranged in the manner that the V-shape bent section is connected to the culture chamber on one side;
the one side of the V-shape bent section connected to the culture chamber has a plurality of pores disposed thereon; and
the inner surface of the V-shape bent section of each side channel is coated with a hydrophobic material while the inner surface of the rest of the side channel is coated with a hydrophilic material;
two upstream reservoirs respectively shaped as funnels and connected to two upstream ends of the first and second side channels; and
two downstream reservoirs respectively shaped as funnels and connected to two downstream ends of the first and second side channels.

2. The chip of claim 1, wherein the inflow channel has an end shaped as a trapezoid toward the loading chamber and connected thereto.

3. The chip of claim 1, further comprising at least one obstructing block disposed within the at least one outflow channel and proximal to the culture chamber.

4. The chip of claim 3, wherein the obstructing block has a size smaller than the size of the 3D tissue.

5. The chip of claim 1, wherein the at least one outflow channel has a section proximal to the culture chamber with its inner dimension being smaller than that of the rest of the outflow channel.

6. The chip of claim 5, wherein the inner dimension of the section is smaller than the diameter or maximal length of the 3D tissue.

7. The chip of claim 1, wherein each of the pores disposed on the one side of the V-shape bent section has a size ranging from 30 µm to 100 µm.

8. The chip of claim 1, wherein
the hydrophobic material is selected from the group consisting of polyepoxide, polyethylene (PE), polystyrene (PS), polyvinylchloride (PVC), polytetrafluorethylene (PTFE), polydimethylsiloxane (PDMS), polyester, polymethyl methacrylate (PMMA), polyurethane (PU), and a combination thereof; and
the hydrophilic material is selected from the group consisting of polyethylene glycol (PEG), oligoethylene glycol (OEG), polyacrylamide (PAM), polyacrylic acid (PAA), polyvinyl alcohol (PVA), polystyrene sulfonic acid (PSS), poly-lysine, and a combination thereof.

9. The chip of claim 1, wherein the culture chamber is made of PMMA, PS, PE, PVC, PU, PAM, polypropylene (PP), polyethylene terephthalate (PET), or a combination thereof.

10. A system for producing a vascularized tissue from a tissue mixture comprising a three-dimensional (3D) tissue, an endothelial cell, a stromal cell, and a hydrogel; the system comprises,
a tissue plate comprising:
a pair of the chips according to claim 1; and
a linking portion disposed between the pair of chips; and
a driving device comprising
a rod having two ends;
an overtube mounted on one end of the rod and has a lumen for the tissue plate to pass therethrough;
a plurality of fastening elements inserted into the overtube and coupled to the linking portion of the tissue plate so as to removably fasten the tissue plate to the overtube; and
a driving element coupled to the other end of the rod for driving the tissue plate to rotate along the longitudinal axis of the rod.

11. A method of producing a vascularized tissue by using the chip of claim 1 comprising,
(a) adding a tissue mixture into the loading chamber, wherein the tissue mixture comprises a three-dimensional (3D) tissue, an endothelial cell, a stromal cell, and a hydrogel;
(b) adding an equal amount of a first medium into each of the two upstream reservoirs, wherein the first medium is free of vascular endothelial growth factor (VEGF) and basic fibroblast growth factor (bFGF);
(c) incubating the product of step (b) at 37°C for at least 3 days;
(d) adding an equal amount of the endothelial cell to each of the two upstream reservoirs;
(e) incubating the product of step (d) at 37°C for at 3 to 6 hours; and
(f) adding an equal amount of a second medium into each of the two upstream reservoirs, wherein the second medium comprises the VEGF and bFGF.

12. The method of claim 11, wherein in step (a), the 3D tissue is a biological sample isolated and dissected from a disease tissue or healthy tissue of a subject.

13. The method of claim 12, wherein the 3D tissue is from a patient-derived xenograft.

14. The method of claim 11, wherein in step (a),
the endothelial cell is a human umbilical vein endothelial cell (HUVEC);
the stromal cell is a fibroblast; and
the hydrogel is a fibrin gel.

15. The method of claim 11, wherein the volume ratio of the 3D tissue and the culture chamber is about 1 : 10 to 1 : 40.
